(19) Europäisches Patentamt / European Patent Office / Office européen des brevets

(11) **EP 2 740 499 B1**

(12) **EUROPEAN PATENT SPECIFICATION**

(45) Date of publication and mention
of the grant of the patent:
**26.09.2018  Bulletin 2018/39**

(51) Int Cl.:
*A61L 31/00* (2006.01)  *A61K 31/047* (2006.01)
*A61L 15/00* (2006.01)  *A61P 41/00* (2006.01)

(21) Application number: **12820730.5**

(22) Date of filing: **30.07.2012**

(86) International application number:
**PCT/JP2012/069326**

(87) International publication number:
**WO 2013/018759 (07.02.2013 Gazette 2013/06)**

(54) **ANTI-ADHESION MEDICAL MATERIAL AND METHOD FOR PRODUCING SAME**

ADHÄSIONSVERHINDERNDE MEDIZINISCHES MATERIAL UND VERFAHREN ZU SEINER HERSTELLUNG

MATÉRIAU MÉDICAL ANTI-ADHÉSION ET PROCÉDÉ DE PRODUCTION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **02.08.2011  JP 2011169585**

(43) Date of publication of application:
**11.06.2014  Bulletin 2014/24**

(73) Proprietor: **Dainichiseika Color & Chemicals Mfg. Co., Ltd.**
**Chuo-ku**
**Tokyo 103-8383 (JP)**

(72) Inventors:
• **NOISHIKI Yasuharu**
**Yokohama-shi**
**Kanagawa 236-0005 (JP)**
• **SANNAN Takanori**
**Tokyo 103-8383 (JP)**
• **ISONO Yasuyuki**
**Tokyo 103-8383 (JP)**
• **KANAO Shinzo**
**Tokyo 103-8383 (JP)**
• **ITOH Hiroshi**
**Tokyo 103-8383 (JP)**
• **IIJIMA Yoshihiko**
**Tokyo 103-8383 (JP)**

(74) Representative: **Blodig, Wolfgang**
**Wächtershäuser & Hartz**
**Patentanwaltspartnerschaft mbB**
**Weinstrasse 8**
**80333 München (DE)**

(56) References cited:
**EP-A2- 1 120 428      WO-A1-98/08550**
**JP-A- 10 085 318      JP-A- 2002 515 086**

• **Y. NOISHIKI ET AL: "Anti-adhesive Membrane for Pleural Cavity", ARTIFICIAL ORGANS, vol. 34, no. 3, 2010, pages 224-229, XP002738458,**
• **JACKSON J K ET AL: "PACLITAXEL-LOADED CROSSLINKED HYALURONIC ACID FILMS FOR THE PREVENTION OF POSTSURGICAL ADHESIONS", PHARMACEUTICAL RESEARCH, vol. 19, no. 4, 1 April 2002 (2002-04-01), pages 411-417, XP009025869, SPRINGER NEW YORK LLC, US ISSN: 0724-8741, DOI: 10.1023/A:1015175108183**
• **DATABASE WPI Week 200870 Thomson Scientific, London, GB; AN 2008-L92445 XP002738487, & JP 2008 155014 A (NICEM LTD) 10 July 2008 (2008-07-10) -& DATABASE EPODOC [Online] EUROPEAN PATENT OFFICE, THE HAGUE, NL; XP002738488, Database accession no. JP-2007308635-A & JP 2008 155014 A (NICEM LTD) 10 July 2008 (2008-07-10)**

**Description**

**Technical Field**

This invention relates to an adhesion preventing medical material capable of preventing bonding, in other words, adhesions which may occur between a wound area and its surrounding tissues or between organs supposed to be separated from each other under normal conditions, and also to a production process thereof.

**Background Art**

The formation of adhesions after operations (post-operative adhesions) is known to take place in many cases to cause various morbid conditions such as intestinal obstruction, infertility, abdominal pain and pelvic pain (see, for example, Non-patent Document 1) . In general, the repair of a wound occurs after an operation, whereby the intended objective, i.e., the treatment by the operation is achieved. On the other hand, the performance of an operation may induce new bonding, in other words, adhesions between organs which are supposed to be separated from each other under normal conditions. The induction of such adhesions may often cause a serious problem such as intestinal obstruction or infertility. When a need arises for a reoperation, the reoperation needs to be started from separating adhesions if the adhesions have been caused by the previous operation. This places an enormous load on the patient and his or her medical staff. If the operative site is located in the thorax or the like, the division of adhesions may be found difficult in many cases. Accordingly, the prevention of post-operative adhesions is an important issue in medical practice, leading to an outstanding desire for the establishment of a safe and reliable measure for the prevention of adhesions.

Under such circumstances as described above, various measures have heretofore been taken, including contrivance relating to surgical procedures, the administration of post-operative adjuvants, and the use of adhesion preventing materials. Of these measures, the administration of post-operative adjuvants and the use of adhesion preventing materials are expected to play a role as an effective auxiliary means. However, the administration of adjuvants involves various problems such that (1) the availability of adhesion preventing effect is not certain, (2) a delay may occur in the healing of a wound, and (3) adhesions may be caused conversely. Technological developments on adjuvants can, therefore, be considered to be practically in stagnation.

In contrast, adhesion preventing materials have relatively good adhesion preventing effect, and are therefore expected to find clinical utility. An adhesion preventing material is a material that is primarily implanted in the vicinity of a wound site to achieve the prevention of adhesions. A foreign body reaction, for example, such as encapsulation by body tissues, however, takes place if an implanted adhesion preventing material remains for a long period of time in the body. As a result, the thus-formed capsules may act, for example, as a cause of adhesions. There is hence a possibility that a problem caused by the adhesion preventing medical material may arise. It is, therefore, considered desirable to use an adhesion preventing material formed of a base material which has biodegradability and requires no surgical extraction.

At present, the adhesion preventing material (adhesion barrier) produced by Genzyme Corporation is widely used in clinical practice. This adhesion preventing material is formed of a polyanionic, hydrophilic biodegradable polymer obtained by crosslinking hyaluronic acid and carboxymethylcellulose (CMC) with a carbodiimide compound, and is marketed under the name of "Seprafilm (registered trademark)". This adhesion preventing material is a product intended for the prevention of post-operative adhesions in the abdominal part and gynecological region. This adhesion preventing material has been observed to exhibit reliable adhesion preventing effect in organs, such as the abdominal part, that perform peristaltic action. No adhesion preventing effect can, however, be expected at sites where a reduction in adhesion by peristaltic action cannot be expected. No adhesion preventing effect can be confirmed even when this adhesion preventing material is actually used, for example, in the thoracic surgery field in an animal experimentation.

Conventional adhesion preventing materials are known to be roughly divided into the following three types:

(1) those to be inserted as a physical barrier to prevent adhesions,
(2) those provided themselves with a property to reject cells for the prevention of adhesions, and
(3) those capable of preventing adhesions by a substance having effectiveness for the prevention of adhesions.

The adhesion preventing materials of the type (1) or (2) have difficulties in achieving absolute prevention of adhesions and are hardly considered to exhibit satisfactory performance, because *inter alia* they are limited in the sites where they can prevent adhesions or have a problem in the compatibility of themselves with the body.

On the other hand, the adhesion preventing materials of the type (3) include those having relatively promising performance. There are known, for example, adhesion preventing materials containing a liposome-mediated non-steroidal anti-inflammatory agent, inhibitor for active oxygen species, retinoid derivative, halofuginone, plasminogen, synthesis or secretion promoter for plasminogen activators, protease produced from a specific microorganism, cyclopropane carboxamide compound, serum albumin, heparin, methionine subjected to oxidation treatment with heparin, leucine, polyhydric alcohol or the like.

As an adhesion preventing material making use of a polyhydric alcohol, an adhesion preventing material with 40 wt% or more of a polyhydric alcohol incorporated in a biocompatible base material has been disclosed (see Patent Document 1). This adhesion preventing material is described to be able to surely prevent adhesions not only in the abdominal part or pelvic part as a subject of the conventional adhesion prevention (technologies) but also in any tissue or at any site. It is to be noted that examples using glycerol as a preferred example of the polyhydric alcohol are disclosed in Patent Document 1.

The use of the adhesion preventing material described in Patent Document 1 makes it possible to effectively achieve its primary object, that is, the prevention of adhesions. The use of a polyhydric alcohol, such as glycerol, having water absorbability may, however, raise a new problem due to the properties of the polyhydric alcohol itself. In the clinical administration of glycerol, for example, hemolysis may occur if high-concentration glycerol enters a blood vessel. It is, therefore, suggested to limit the administration rate and/or administration interval of glycerol for the prevention of hemolysis (see Non-patent Document 2). Through basic studies using experimental animals, it has become evident that hemolysis occurs when a glycerol solution of 50 wt.% concentration (glycerol enema solution) flows into blood from a damaged site or the like of the rectal mucosa (see Non-patent Documents 3 and 4). Further, glycerol is also concerned to lead to the onset of hemolysis-induced renal failure.

A composition with glycerol similarly incorporated as a plasticizer in a biocompatible crosslinked hydrogel the equilibrium swell of which is in a specific range is also disclosed for the prevention or suppression of tissue adhesions (for example, adhesions of spinal tissues) after operations or the like (see Patent Document 2). In addition, an adhesion preventing material with glycerol added to a material formed from a UV-crosslinked gelatin is also disclosed (see Patent Document 3). With a view to enhancing the adhesiveness to the body, the adhesion preventing material described in Patent Document 3 is improved in softness and pliability (suppleness) by the addition of glycerol.

An adhesion preventing material with glycerol incorporated as a suppleness improving and moisturizing agent in a large quantity is brought into a high water-content state after implanted in vivo, because the glycerol absorbs surrounding water. The adhesion preventing material, which has been brought into the high-water content state, is known to induce a state of edema in a tissue and hence to raise such a problem that the healing of the tissue is delayed or the tissue is left unvulnerable to bacterial infection (see Patent Document 4). According to Patent Document 4, the concentration of glycerol is described to be desirously set at 20% or lower based on the dry weight of the medical material including the glycerol.

**Prior Art Documents**

**Patent Documents**

Patent Document 1: JP-A-2008-155014
Patent Document 2: JP-A-2006-231090
Patent Document 3: JP-A-2000-37450
Patent Document 4: JP-A-2010-213984

**Non-patent Documents**

Non-patent Document 1: FUJISHITA, Akira, YOSHIDA, Shiko, SIMOMURA, Tomoko, MATSUMOTO, Ayumi: "An Overview of Adhesion Preventing Methods and Adhesion Preventing Measures - Centering around Gynecology-related Literature", OBSTERICAL AND GYNECOLOGICAL PRACTICE, 59(8), 1159-1167 (2010)
Non-patent Document 2: SUGIHARA, Hisashi: "Erythrocyte Hemolysis by Glycerol", THE JAPANESE JOURNAL of CLINICAL HEMATOLOGY, 24(8), 1012-1019 (1983)
Non-patent Document 3: TAKEDA, Toshiaki, ISHIDA, Yoko, KAWASHIMA, Midori: "Experimental Study on Relationship between Glycerol Enema Solution and Hemolysis in Rats", JOURNAL OF JAPANESE SOCIETY OF NURSING RESEARCH, 26(4), 81-88 (2003)
Non-patent Document 4: TAKEDA, Toshiaki: "Verification Study on Induction of Hemolysis by Glycerol Enema in Experimental Animals", JAPANESE JOURNAL OF NURSING ART AND SCIENCE, 5(1), 45-50 (2006)

Yasuharu Noishiki et al. "Anti-adhesive Membrane for Pleural Cavity", Artificial Organs, Vol. 32, No. 3, 2010, 224-229 (XP002738458) discloses an anti-adhesive membrane for the pleural cavity comprising high amounts of glycerol.

## Disclosure of the Invention

### Problem to Be Solved by the Invention

When a medical material with a polyhydric alcohol such as glycerol contained therein is used, water is caused to gather around the medical material under the osmotic pressure of the polyhydric alcohol so that a water layer is formed. As it is extremely difficult for cells to invade a water layer formed as described above, the incorporation of such a polyhydric alcohol makes it possible to provide a medical material that can surely prevent adhesions in any tissue or at any site. Even if an improvement in suppleness is an object, the use of a medical material with a polyhydric alcohol contained at a high concentration therein, however, leads to the actualization of such a clinical problem that an adverse event such as intra-tissue edema or hemolysis becomes liable to occur.

As mentioned above, cells can hardly invade a water layer formed as a consequence of the existence of a polyhydric alcohol, and therefore adhesions are prevented. On the other hand, there is a problem that the degradation and resorption rates of a base material in the body are reduced. There is, accordingly, an outstanding desire for the development of a medical material, which can prevent adhesions, hardly causes an adverse event such as intra-tissue edema, and is promptly degraded and resorbed after the prevention of adhesions.

The present invention has been completed in view of such problems of the conventional technologies. Objects of the present invention are, therefore, to provide an adhesion preventing medical material, which can surely prevent post-operative adhesions in various tissues or at various sites, hardly causes an adverse event such as intra-tissue edema or hemolysis or an adverse event as a consequence of remaining of a base material in the body, and is promptly degraded and resorbed after the prevention of adhesions, and also a production process thereof.

### Means for Solving the Problem

The present inventors conducted enthusiastic research to solve the above-described problems, and as a result, found that the use of a bioresorbable base material, which satisfies such specific conditions as allowing a large majority of a polyhydric alcohol incorporated inside the bioresorbable base material to be promptly released after implanted, brings about sufficient effect for the prevention of adhesions, hardly gives adverse effect on the surrounding tissues of an implanted site, and is promptly degraded and resorbed in vivo after the release of the polyhydric alcohol, leading to the completion of the present invention.

Described specifically, the following adhesion preventing medical materials are provided according to the present invention.

[1] An adhesion preventing medical material comprising a bioresorbable base material and an aqueous polyhydric alcohol solution, which contains the polyhydric alcohol, held in said bioresorbable base material, said bioresorbable base material comprising a bioresorbable material and having a swelling degree of 200 to 3,000 mass% and a water elution rate of not higher than 10 mass%, wherein, after immersed for 3 hours in water of 25°C in an amount at least 50 times a total mass of the aqueous polyhydric alcohol solution and the bioresorbable base material, the polyhydric alcohol remains in an amount of not greater than 30 mass% of that of the polyhydric alcohol before the immersion, wherein the bioresorbable material is at least one bioresorbable material selected from the group consisting of gelatin, collagen, chitin, partially-deacetylated chitin, chitosan, hyaluronic acid, carboxymethylcellulose, derivatives thereof, and salts thereof, wherein the proportion of the polyhydric alcohol (the content ratio of the polyhydric alcohol) to the mass of the bioresorbable base material is 10 mass% or greater, and wherein the initial concentration of a polyhydric alcohol, namely the concentration of an aqueous solution of the polyhydric alcohol contained in the adhesion preventing medical material is in a range of 1 to 20 wt%.

[2] The adhesion preventing medical material as described above in [1], wherein the polyhydric alcohol is glycerol, and the glycerol amounts to a proportion of not greater than 70 mass% of a mass of the bioresorbable base material.

[3] The adhesion preventing medical material as described above in [1] or [2], wherein the bioresorbable material is at least one bioresorbable material selected from the group consisting of gelatin, collagen, chitin, partially-deacetylated chitin, chitosan, hyaluronic acid, carboxymethylcellulose, derivatives thereof, and salts thereof.

[4] The adhesion preventing medical material as described above in any one of [1] -[3], wherein the bioresorbable base material is in a form of a film, spheres, a string, a rod, a plate, a tube, rectangles, a powder, a colloid, a liquid or a sponge.

[5] The adhesion preventing medical material as described above in any one of [1] -[4], which has an in vivo shape retention time (in days) (T) of 1<T<21.

According to the present invention, the following processes are also provided for the production of the adhesion preventing medical materials.

[6] A production process of the adhesion preventing medical material as described above in any one of [1] -[5], comprising the following step: holding, in the bioresorbable base material, the aqueous polyhydric alcohol solution with the polyhydric alcohol contained therein.

[7] The production process as described above in [6], wherein the aqueous polyhydric alcohol solution is held after subjecting the bioresorbable base material to water insolubilization treatment.

[8] The production process as described above in [6] , wherein the bioresorbable base material with the polyhydric alcohol contained therein is subjected to water insolubilization treatment.

[9] The production process as described above in [6], wherein, when the bioresorbable material is a salt that dissolves in a neutral range, the bioresorbable base material is subjected to water insolubilization treatment by adjusting its pH before or after the adhesion preventing medical material is processed into a predetermined form.

[10] The production process as described above in [7], wherein the bioresorbable base material is subjected to water insolubilization treatment by irradiation of radiation.

[11] The production process as described above in [10], wherein the radiation is irradiated to 10 to 60 kGy.

[12] The production process as described above in [7] or [8], wherein the bioresorbable base material is subjected to water insolubilization treatment with polyvalent cations.

[13] The production process as described above in [12], wherein the polyvalent cations are at least one type of polyvalent cations selected from a group consisting of calcium ions, titanium ions, magnesium ions, iron ions, and zirconium ions.

[14] The production process as described above in [7] or [8], wherein the bioresorbable base material is subjected to water insolubilization treatment with a compound that has at least two functional groups in a molecule thereof.

[15] The production process as described above in any one of [6] -[14], further comprising the following step: lyophilizing an aqueous solution of the bioresorbable material to obtain the bioresorbable base material in a sponge form.

**Advantageous Effects of the Invention**

With the adhesion preventing medical material according to the present invention, the released polyhydric alcohol attracts water from peripheries of an implanted site to form a water layer around the implanted site, and owing to the water layer so formed, the prevention of adhesions is effectively achieved in various tissues or at various sites. Further, a large majority of the polyhydric alcohol is promptly released after implanted, so that the polyhydric alcohol is prevented from being released over a long term. An adverse event such as intra-tissue edema or hemolysis hardly occurs accordingly. Moreover, the bioresorbable base material is promptly degraded and resorbed in the body after the release of the polyhydric alcohol, and therefore, an adverse event as a consequence of remaining of the base material in the body hardly occurs.

According to the production process of the present invention, it is possible to produce an adhesion preventing medical material, which can surely prevent post-operative adhesions in various tissues or at various sites, hardly causes an adverse event such as intra-tissue edema or hemolysis or an adverse event as a consequence of remaining of the base material in the body, and is promptly degraded and resorbed after the prevention of adhesions.

**Modes for Carrying out the Invention**

The adhesion preventing medical material according to the present invention comprises a bioresorbable base material and an aqueous polyhydric alcohol solution, which contains the polyhydric alcohol, held in said bioresorbable base material. The bioresorbable base material comprises a bioresorbable material. Further, after immersed for 3 hours in water of 25°C in an amount at least 50 times the total mass of the aqueous polyhydric alcohol solution and the bioresorbable base material, the polyhydric alcohol remains in an amount of not greater than 30 mass% of that of the polyhydric alcohol before the immersion. Based on preferred embodiments, the adhesion preventing medical material according to the present invention will hereinafter be described in detail.

The polyhydric alcohol is a component that exhibits an adhesion preventing function. No particular limitation is imposed on the polyhydric alcohol insofar as it does not give much load on the living body and is allowed to promptly spread around an implanted site. Specific examples of the polyhydric alcohol include ethylene glycol, diethylene glycol, polyethylene glycol, methyl glycerol, polyoxyethylene glycoside, maltitol, mannitol, xylitol, sorbitol, reducing starch syrup, dipropylene glycol, butylene glycol, propylene glycol, glycerol(glycerin), polyglycerol, glycerol fatty acid esters, and the like. Among these, polyhydric alcohols employed in the medical field and food field, such as glycerol, xylitol, sorbitol and low-molecular polyethylene glycol, can be suitably used. These suitably usable polyhydric alcohols can be obtained from the market, and can be used as they are. As to glycerol, sorbitol and the like, those complying with the Japanese Pharmacopoeia are desirably used. Glycerol is particularly preferred, because it is a material the safety of which is so high that it is also used as an injection into the vein.

The present inventors conducted an enthusiastic study on the prevention of adhesions with this safe material. As a result, it was found that, when a large majority of a polyhydric alcohol contained in an adhesion preventing medical material (implant material) is promptly released, specifically when the remaining percentage of the polyhydric alcohol after held for 3 hours in water is 30 mass% or less, water is attracted from peripheries of a wound part to form a water layer around the wound part and cells are inhibited from migrating (invading) from peripheries of the thus-formed water layer to achieve the prevention of adhesions.

A polyhydric alcohol has water absorbability, so that an adverse event such as intra-tissue edema may be induced as a consequence of this water absorbability when the polyhydric alcohol exists at a high concentration in the body. It was found that, when the initial concentration of a polyhydric alcohol, namely the concentration of an aqueous solution of a polyhydric alcohol contained in an implant material is in a range of 1 to 20 wt%, the polyhydric alcohol is prevented from being released over a long term and the concentration of the aqueous solution of the polyhydric alcohol, which exists in tissues around the implant material, is also maintained at such a low concentration as causing no problem, thereby making it possible to avoid an adverse event which would otherwise be caused by the polyhydric alcohol. It is to be noted that, when a polyhydric alcohol is contained in an adhesion preventing medical material, the concentration of an aqueous solution of the polyhydric alcohol as formed after swelling of the adhesion preventing medical material as a consequence of absorption of water is only required to fall within the above-described concentration range.

If the adhesion preventing medical material remains over a long term in the body after the release of the polyhydric alcohol, various events such as the formation of capsules by in vivo tissues are expected to occur. As a countermeasure for such a potential problem, the present inventors use a bioresorbable material as a constituent material of a base material for the adhesion preventing medical material. Described specifically, in an initial stage of implant placement that the polyhydric alcohol is contained in the base material, the polyhydric alcohol inhibits the degradation of the base material. The base material, therefore, remains at the implanted site and releases the polyhydric alcohol, thereby contributing to the prevention of adhesions. Subsequent to the release of the polyhydric alcohol from the base material, on the other hand, the base material is promptly degraded and resorbed in the body. It was found that owing to the above-described mechanism, the provision of an adhesion preventing medical material, which does not bring about such adverse effect that would otherwise be caused due to the remaining of the base material in the body and which has high safety, can be realized. This finding has lead to the completion of the present invention.

In approximately three days after implanted, the adhesion preventing medical material according to the present invention generally allows most of the polyhydric alcohol to be released after implanted, and the prevention of adhesions at the site of a wound can be achieved. Such a release rate of the polyhydric alcohol is substantially equivalent in vitro to the fact that after immersed for 3 hours in water of 25°C in an amount at least 50 times the total mass of the aqueous polyhydric alcohol solution and the bioresorbable base material, the polyhydric alcohol remains in an amount of not greater than 30 mass% of that of the polyhydric alcohol before the immersion. This remaining amount of the polyhydric alcohol as measured in vitro may hereinafter be also referred to as "the retention rate (mass%) of the polyhydric alcohol". This "retention rate (A(%)) of the polyhydric alcohol" can be calculated by the following equation (1). It is to be noted that in the following equation (1), letter A represents the retention rate (%) of the polyhydric alcohol, letter B denotes the remaining amount (g) of the polyhydric alcohol after immersed for 3 hours in water of 25°C in an amount at least 50 times the total mass of the aqueous polyhydric alcohol solution and the bioresorbable base material, and letter C means the amount (g) of the polyhydric alcohol before immersed.

$$A\ (\%)\ =\ (B/C)\ \times\ 100\ \ \ \ldots\ldots\ldots\ (1)$$

The safe and effective prevention of adhesions can be achieved insofar as such a retention rate of the polyhydric alcohol as described above is realized and the polyhydric alcohol is released in a sufficiently short time. If the retention rate of the polyhydric alcohol exceeds 30 mass%, on the other hand, the degradation and resorption of the bioresorbable base material is inhibited by the remaining polyhydric alcohol so that the bioresorbable base material tends to become liable to remain in the body. It is known that the degradation of the bioresorbable base material by hyaluronidase (bioresorbable material: hyaluronic acid), chitinase or lysozyme (bioresorbable material: chitin), chitosanase or lysozyme (bioresorbable material: chitosan), or a protease such as collagenase (bioresorbable material: collagen) or trypsin (bioresorbable material: gelatin) is inhibited, and therefore, the bioresorbable base material becomes liable to remain in the body.

The retention rate of the polyhydric alcohol can be measured by a method to be described hereinafter. Described specifically, a piece of a sample of an adhesion preventing medical material is placed in water in an amount at least 50 times the total mass of an aqueous polyhydric alcohol solution and a bioresorbable base material, and is left over at 25°C for 3 hours. Three hours later, the piece of the sample is taken out, and the concentration of the polyhydric alcohol in the water is measured by HPLC analysis or the like. From the concentration of the polyhydric alcohol so measured, the total amount of the polyhydric alcohol in the water is calculated, and further, the amount of the polyhydric acid held (remaining) in the piece of the sample is calculated.

The retention rate of the polyhydric alcohol can be suitably adjusted by changing the swelling degree of the bioresorbable base material. When the swelling degree of the bioresorbable base material is high (the density of the bioresorbable base material is low), for example, the release rate of the polyhydric alcohol increases so that the retention rate of the polyhydric alcohol decreases. It is to be noted that an excessively high swelling degree of the bioresorbable base material makes it difficult to allow a water layer to remain for a sufficient period of time around an implanted site after implanted, and therefore, leads to insufficient adhesion preventing effect. When the swelling degree of the bioresorbable base material is low (the density of the bioresorbable base material is high), on the other hand, the release rate of the polyhydric alcohol decreases so that the retention rate of the polyhydric alcohol increases. It is to be noted that an unduly low swelling degree of the bioresorbable base material allows the polyhydric alcohol to remain for a long period of time in the bioresorbable base material, and therefore, the degradation and resorption of the bioresorbable base material is delayed. With the foregoing in view, the swelling degree of the bioresorbable base material, which makes up the adhesion preventing medical material according to the present invention, is 200 to 3, 000 mass% , with 500 to 2,000 mass% being preferred.

The term "swelling degree" as used herein means the proportion (mass%) of "the mass of the bioresorbable base material after held in water (after swelling)" to "the mass of the bioresorbable base material before held in water (before swelling)". The swelling degree of the bioresorbable base material can be adjusted, for example, by controlling conditions for the production of the bioresorbable base material. Described specifically, when the bioresorbable material is gelatin, the degree of the resulting bioresorbable base material can be adjusted to the above-described numerical value range by controlling the temperature (drying temperature) upon drying an aqueous solution of the gelatin to obtain the (pre-swelling) bioresorbable base material in a predetermined form or by controlling the extent of crosslinking treatment. When the bioresorbable material is sodium hyaluronate, the swelling degree of the resulting bioresorbable base material can be adjusted to the above-described numerical value range by controlling the concentration of sodium hyaluronate or the extent of water insolubilization treatment (crosslinking treatment). It is to be noted that for the adjustment of the extent of the water insolubilization treatment (crosslinking treatment), it is only necessary, for example, to control the concentration of polyvalent cations, such as calcium ions, titanium ions, magnesium ions, iron ions or zirconium ions, to be used.

The retention rate of the polyhydric alcohol can also be suitably adjusted by changing the water elution rate of the bioresorbable base material. When the water elution rate is low, for example, the swelling degree decreases so that the retention rate of the polyhydric alcohol increases . When the water elution rate is high, on the other hand, the swelling degree increases so that the retention rate of the polyhydric alcohol decreases. With the foregoing in view, the water retention rate of the bioresorbable base material, which makes up the adhesion preventing medical material according to the present invention, is not higher than 10 mass%, with 2 to 10 mass% being preferred. It is to be noted that a water retention rate higher than 10 mass% makes it difficult to form, by the polyhydric alcohol, a water layer around the implant material. If the water retention rate is lower than 2 mass%, on the other hand, the adhesiveness of the implant material to an organ is reduced, thereby possibly making it difficult to arrange the implant material at a desired site.

It is to be noted that the term "water retention rate" as used herein means the proportion (mass%) of "the mass of the bioresorbable base material eluted in water after holding" to "the mass of the bioresorbable base material before holding" when the bioresorbable base material is held in water at 40°C for 20 minutes. The water retention rate of the bioresorbable base material can be suitably adjusted, for example, by changing the crosslinking degree of the bioresorbable material that makes up the bioresorbable base material.

As mentioned above, the bioresorbable base material, which makes up the adhesion preventing medical material according to the present invention, is specified in swelling degree and water elution rate to the above-described predetermined numerical ranges. In other words, the bioresorbable base material is designed such that a majority of the polyhydric alcohol incorporated inside the base material is promptly released after implanted. As the polyhydric alcohol is hence promptly released even when it is contained at a relatively high concentration, an adverse event such as intra-tissue edema or hemolysis hardly occurs, and after the prevention of adhesions, the bioresorbable base material is promptly degraded and resorbed. With the foregoing in view, the proportion of the polyhydric alcohol (the content ratio of the polyhydric alcohol) to the mass of the bioresorbable base material may be preferably 70 mass% or less, more preferably 40 mass% or less, particularly preferably 30 mass% or less. A content ratio of the polyhydric alcohol greater than 70 mass% leads to an increased possibility of the occurrence of an adverse event such as intra-tissue edema or hemolysis. An unduly small content ratio of the polyhydric alcohol, on the other hand, may fail to obtain sufficient adhesion preventing effect. Accordingly, the content ratio of the polyhydric alcohol is 10 mass% or greater, more preferably 20 mass% or greater. It is to be noted that the expression "the mass of the bioresorbable base material", which serves as a base for calculating "the content ratio of the polyhydric alcohol" herein, means the net mass of the bioresorbable base material itself (the mass of the bioresorbable base material) without the polyhydric alcohol or the aqueous polyhydric alcohol solution and the like.

The bioresorbable base material comprises the bioresorbable material, and serves as a skeleton of the adhesion preventing medical material. The bioresorbable material may be either a naturally occurring polymer material or a synthetic polymer material (not forming part of the invention), insofar as it is a component that is degradable and resorbable in the body. Concerning the naturally occurring polymer material, no limitation is imposed on its origin, collection method or the like, including the formation of the material by a recombinant method. It is to be noted that the proportion of the bioresorbable material contained in the bioresorbable base material may be preferably 70 mass% or greater, more preferably 90 mass% or greater based on the mass of the bioresorbable base material (before swelling).

Specific examples of the naturally occurring polymer material include naturally occurring polysaccharides such as hyaluronic acid, chitin, partially-deacetylated chitin, chitosan, dextran (not forming part of the invention), alginic acid (not forming part of the invention), pullulan (not forming part of the invention), cellulose (not forming part of the invention), carboxymethylcellulose (CMC), carboxyethylcellulose (not forming part of the invention), starch (not forming part of the invention), amylose (not forming part of the invention), and amylopectin (not forming part of the invention), derivatives thereof, salts thereof, and degradation products thereof (not forming part of the invention), the proteins collagen and gelatin, and derivatives thereof. The at least one naturally occurring polymer material is selected from the group consisting of gelatin, collagen, chitin, partially-deacetylated chitin, chitosan, hyaluronic acid, CMC, derivatives thereof, and salts thereof. These naturally occurring polymer materials can be obtained from the market, or those synthesized may also be used as needed. It is to be noted that concerning gelatin, hyaluronic acid and carboxymethylcellulose (CMC), those complying with the Japanese Pharmacopoeia may be used desirably.

Specific examples of the synthetic polymer material (not forming part of the invention) include polyvinyl alcohol, polyethylene glycol, dioxanone, polycaproic acid, polyarachidonic acid, polymethylene carbonate, trimethylene carbonate, derivatives thereof, salts thereof, and copolymers thereof; inorganic-organic hybrid materials such as polyphosphazene; and the like. These synthetic polymer materials can be obtained from the market, or those synthesized may also be used as needed.

The preferred numerical value range of the swelling degree of the bioresorbable base material differs a little depending on the manner of use, form and the like of the adhesion preventing medical material. Described specifically, when the adhesion preventing medical material is used while maintaining its form, for example, when the adhesion preventing medical material is held by tweezers and is placed on or wound around an application site, the swelling degree of the bioresorbable base material may be set preferably at 200 to 1,000 mass%. On the other hand, when the adhesion preventing medical material is placed on an application site by using a removable support such as paper without directly holding it or when the adhesion preventing medical material is in a particulate form and is used by spraying or similarly applying same, the swelling degree may be set preferably at 1,000 to 3,000 mass% to enhance the adhesiveness.

No particular limitation is imposed on the form (shape) of the bioresorbable base material. Examples of preferred forms of the bioresorbable base material include film form, sphere form, string form, rod form, plate form, tube form, rectangle form, powder form, colloid form, liquid form, sponge form and the like. The more preferred form of the bioresorbable base material differs depending on the application site (operative site). In the case of those having a thickness out of these forms, the thickness may preferably range from 10 to 2,000 $\mu$m. A thickness smaller than 10 $\mu$m tends to result in an insufficient function as a physical barrier, while a thickness greater than 2,000 $\mu$m tends to become an obstacle to the maintenance of the function of a body tissue. When the bioresorbable base material is in a film form, the bioresorbable base material can be formed into a composite adhesion-preventing medical material with a film, which is impervious to the polyhydric alcohol, being arranged on one side of the bioresorbable base material.

The in vivo shape retention time (in days) (T) of the adhesion preventing medical material may be preferably $1<T<28$, more preferably $1<T<21$. It is to be noted that the term "shape retention time (in days)" means the days over which the original shape is retained after implanted in vivo. This means that, even if some detritus of the adhesion preventing medical material is confirmed, the shape retention time has elapsed insofar as the adhesion preventing medical material is not in the original shape but is in the form of discrete fragments. A shape retention time of 1 day or shorter tends to make it difficult to obtain sufficient adhesion preventing effect. On the other hand, a shape retention time of 28 days or longer tends to increase the possibility that an adverse event would be induced as a consequence of remaining in the body. Upon formation of adhesive tissues, a network of fibrin is first formed over a wound as a preceding step. Fibroblasts then invade the thus-formed network to produce collagen fibers, whereby the adhesive tissues begin to be formed. This series of phenomena begins from the 1st day after the operation, and becomes extremely active on or around the 7th day. It is, therefore, desired that the shape of the adhesion preventing medical material is retained for at least one day after which the formation of the network of fibrin and the production of collagen fibers begin. On the other hand, an excessively long shape retention time may raise a problem such as the formation of capsules.

By subjecting the bioresorbable base material to water insolubilization treatment, the shape retention time (in days) of the adhesion preventing medical material can be adjusted. Moreover, the water insolubilization treatment of the bioresorbable base material makes it possible to adjust the water elution rate to the predetermined numerical value range. As a specific method for conducting the water insolubilization treatment, a method that subjects the bioresorbable base material to crosslinking treatment can be mentioned. As the crosslinking treatment, it is possible to mention chemical crosslinking that uses a crosslinking agent; radiation crosslinking that is conducted by irradiating radiation such as ultraviolet rays, $\gamma$ rays or an electron beam; thermal crosslinking by heating; crosslinking by repeated freezing and thawing; or the like. It is to be noted that, when a crosslinking agent is used, there is a need to avoid such inconvenience that the crosslinking agent would remain to produce cytotoxicity.

When a salt that dissolves in a neutral range (pH: 5.0 to 8.0) is used as a bioresorbable material, a water-insolubilized bioresorbable base material can be obtained by adjusting the pH of a solution of the salt before or after processing the salt into a predetermined form such that its salt-form groups are converted to free carboxyl groups, free amino groups, or the like. As the salt that dissolves in the neutral range, a hyaluronate salt, chitosan salt, chondroitin sulfate salt (not forming part of the invention), polylysine salt (not forming part of the invention), alginate salt (not forming part of the invention), or the like can be mentioned. The pH can be adjusted, for example, by treatment with an acid or alkali. The above-mentioned insolubilization methods can be used either singly or as a combination of two or more thereof.

When the bioresorbable base material is hydrophobic, it is preferred to use a biological moisturizing agent that can spread together with the polyhydric alcohol around the adhesion preventing medical material according to the present invention when the adhesion preventing medical material is implanted. The use of the biological moisturizing agent can impart suppleness to the adhesion preventing medical material. Specific examples of the biological moisturizing agent include NMF (natural moisturizing factor), "Aqualizer-EJ", "Prodew", mixed isomerized sugar ("Pentavitin"), amino acids, L-aspartic acid, sodium L-aspartate, DL-alanine, L-arginine, L-isoleucine, lysine hydrochloride (L-lysine hydrochloride), glycine (aminoacetic acid), L-glutamine, L-glutamic acid, sodium L-glutamate, gamma-aminobutyric acid (piperidine), L-threonine, sericin, serine, L-tyrosine, L-triptophan, L-valine, L-histidine hydrochloride, L-hydroxyproline (L-oxyproline), phenylalanine, L-proline, L-leucine, DL-pyrrolidone carboxylic acid (PCA), sodium DL-pyrrolidone carboxylate, lactic acid, sodium lactate, urea, uric acid, acidic muco polysaccharides, extracted liquid from umbilical cord, extracted liquid from crista galli, hyaluronic acid, sodium hyaluronate, sodium chondoroitin sulfate, glucuronic acid, glucuron, collagen, soluble collagen, collagen hydrolyzate (gelatin), atelocollagen, elastin, water-soluble elastin, intermolecular lipid, sphingolipid (ceramide), HS-oil, keratin, hydrolyzed keratin, keratin amino acids, cystine, L-methionine, cystine, nucleic acids, deoxyribonucleic acids (DNA), ribonucleic acids (RNA), guanosine, guanine, phosphoric acid, adenosine triphosphate (ATP), tryptophan adenosine, riboflavin sodium phosphate, phospholipids, lecithin, soybean phospholipid (soybean lecithin), soybean lysophospholipid (lysolecithin), egg yolk lecithin (egg yolk phospholipid), enzymes, plant complex enzymes, protease, lipase, and the like. These biological moisturizing agents can be used either singly or as a combination of two or more thereof. The content of such a biological moisturizing agent is suitably set within a range where suppleness is imparted to the adhesion preventing medical material but the characteristic properties of the adhesion preventing medical material are not inhibited.

The polyhydric alcohol and the biological moisturizing agent, which may be used as needed, are only required to be contained in the bioresorbable base material. As an alternative, however, they may be immobilized on the bioresorbable base material by a chemical or physical means. When the polyhydric alcohol and biological moisturizing agent are immobilized on the bioresorbable base material, the bioresorbable base material continuously absorbs water from surroundings, and at the same time, the polyhydric alcohol and biological moisturizing agent are released out of the adhesion preventing medical material. As a consequence, water is also held outside the adhesion preventing medical material to bring about adhesion preventing effect.

When the adhesion preventing medical material is in the form of a film or plate, it can be used, for example, by inserting same between tissues where the prevention of adhesions is desired. It is to be noted that the size and shape may be adjusted at the time of use. When the adhesion preventing medical material is in the form of spheres or rectangles, it can be used, for example, by filling same in a lost part where the prevention of adhesions is desired. When the adhesion preventing medical material is in the form of a string, it can be used, for example, by winding same around a tubular site where the prevention of adhesions is desired. When the adhesion preventing medical material is in the form of a rod, it can be used, for example, by inserting same into a cavity, stoma or the like where the inhibition of adhesions is desired. When the adhesion preventing medical material is in the form of a tube, it can be used, for example, for a member, such as a drainage tube, which is in a tubular form and is desired to be prevented from adhesions with surroundings. If contrivance is made on the form of the adhesion preventing medical material, spraying is also possible. Described specifically, the adhesion preventing medical material may be sprayed in a fine powder form by using a pressurized container, or the adhesion preventing medical material and polyhydric alcohol may be sprayed in powder forms, respectively, such that they integrate with each other at the time of the spraying.

The adhesion preventing medical material according to the present invention can effectively prevent adhesions even when applied to tissues or the like which do not undergo much peristaltic action and have been hardly prevented from adhesions. The adhesion preventing medical material according to the present invention can also be applied not only to the abdominal part but also to sites, such as the thorax, where the division of adhesions is difficult. Applicable sites can include wound sites in the abdominal cavity, thoracic cavity, skull, pericardium, ventricles, myocardial, blood vessels, liver, eyeball surroundings, eyeballs, lacrimal ducts, norstrils, connective tissues, tendon sheaths, dura mater, marrow, tracheas, bronchi, and the like. By implanting the adhesion preventing medical material according to the present invention in the above-described site, clot adhesion preventing effect and cell attachment preventing effect can also be exhibited in addition to adhesion preventing effect.

A description will hereinafter be made about a prevention mechanism of adhesions, which can be presumed for the adhesion preventing medical material according to the present invention. At a part where adhesions have occurred, the cells at a site where tissues are to be repaired, and phagocytic cells such as macrophages are producing cytokines such as cell growth factors. These cytokines locally accumulate to a high concentration, whereby fibroblasts and the like, which serve to repair the tissues, actively migrate and invade from surroundings. As these cells produce a great deal of collagen fibers, adhesive tissues are promptly formed. For the prevention of adhesions, it is, therefore, necessary (1) to remove cytokines, which have locally accumulated to a high concentration, and to disperse them to normal parts and (2) to suppress the activities of macrophages that invade a wound site. The prevention of adhesions can hence be achieved by allowing a wound site to naturally heal itself while supporting the wound site with the adhesion preventing medical material to keep the wound site apart from surrounding tissues and also proceeding with the removal of cytokines.

When the adhesion preventing medical material according to the present invention is used, the polyhydric alcohol is spread around the adhesion preventing medical material. As a result of mixing of the thus-spread polyhydric alcohol with water, a single water layer is formed around the adhesion preventing medical material. The water layer so formed prevents the invasion of cells from surroundings, and further, is extremely hardly affected by cytokines produced around a fibrin network formed at the wound site. It is, therefore, possible to prevent adhesions by collagen produced at the wound site. When the adhesion preventing medical material according to the present invention is implanted, for example, by inserting it into a space between a wounded site and its surrounding tissues, the adhesion preventing medical material that exists in the space exhibits the above-described behavior, and prevents the wounded site and its surrounding tissues from adhering together via a fibrin network. It is, therefore, possible to prevent the formation of adhesive tissues between the wound area and its surrounding tissues.

A description will hereinafter be made about the process according to the present invention for the production of the adhesion preventing medical material. The production process of the present invention for the adhesion preventing medical material includes a step of holding, in the bioresorbable base material, the aqueous polyhydric alcohol solution with the polyhydric alcohol contained therein. As methods for holding the aqueous polyhydric alcohol solution in the bioresorbable base material, there are (1) a method that has the polyhydric alcohol contained in a raw material upon formation of the bioresorbable base material and produces the adhesion preventing medical material in a dry state (not forming part of the invention), and (2) a method that immerses the bioresorbable base material, which has been processed into a predetermined form, in an aqueous solution of the polyhydric alcohol, said aqueous solution having a predetermined concentration, to produce the adhesion preventing medical material in a wet state.

In the above-described method (1), the polyhydric alcohol is mixed in the raw material that contains the bioresorbable material, and after processing the resultant mixture into a predetermined form, drying is conducted to obtain the bioresorbable base material with the polyhydric alcohol contained therein (not forming part of the invention). In the above-described method (2), on the other hand, the bioresorbable base material, which has been processed into the predetermined form, is immersed in the aqueous polyhydric alcohol solution to purge the interior of the bioresorbable base material with the aqueous polyhydric alcohol solution of the predetermined concentration, so that the aqueous polyhydric alcohol solution of the predetermined concentration is held to obtain the desired adhesion preventing medical material.

To adjust the water elution rate of the bioresorbable base material to the predetermined numerical value range, it is only necessary to subject the bioresorbable base material to water insolubilization treatment. This water insolubilization treatment may be either before having the polyhydric alcohol or the aqueous polyhydric alcohol solution held in the bioresorbable base material or after having the polyhydric alcohol or the aqueous polyhydric alcohol solution held in the bioresorbable base material.

The bioresorbable base material can be water-insolubilized by subjecting the bioresorbable material to crosslinking treatment. As methods for subjecting the bioresorbable base material to water insolubilization treatment, there can be mentioned, for example, a radiation crosslinking method that irradiates radiation to give an irradiance level of 10 to 60 kGy, a chemical crosslinking method that uses a crosslinking agent, and the like. As the crosslinking agent, it is preferred to use a compound having at least two functional groups in a molecule thereof. Specific examples of this "compound having at least two functional groups in a molecule thereof" include aldehyde compounds such as glutaraldehyde, formaldehyde and dialdehyde starch; water-soluble polyfunctional epoxy compounds such as glycerol polyglycidyl ether and polyglycerol polyglycidyl ether; water-soluble carbodiimide compounds such as 1-ethyl-3-(3-dimethylaminopropyl)carbodiimide, cyclohexyl-3-(2-morpholinoethyl)carbodiimide and dicyclohexylcarbodiimide; isocyanate compounds such as hexamethylene diisocyanate; and the like. These crosslinking agents can be used either singly or as a combination of two or more thereof. Depending on the kind of the bioresorbable material, the bioresorbable base material can be subjected to water insolubilization treatment with polyvalent cations. As specific examples of the polyvalent cations, calcium ions, titanium ions, magnesium ions, iron ions, zirconium ions, and the like can be mentioned.

To obtain the bioresorbable base material in a sponge form, it is only necessary, for example, to lyophilize an aqueous solution of the bioresorbable material.

**Examples**

Based on examples, the present invention will next be described more specifically. It is to be noted that the designations of "parts" and "%" in the examples are on a mass basis. It is also to be noted that the present invention shall not be limited by these examples.

Measuring method of polyhydric alcohol (glycerol) retention rate

Standard glycerol solutions (aqueous solutions) of varied glycerol concentrations were analyzed by HPLC, and based on peak areas of glycerol, a calibration curve for glycerol was prepared. A test piece (0.5 g) of an adhesion preventing medical material was placed in water (30 mL) of 20°C, and was left over for 3 hours. After the test piece was taken out of the water, a fraction (1 mL) of the water was sampled and was subjected to an HPLC analysis. The concentration of glycerol was determined from the calibration curve prepared as described above, and the glycerol retention rate was calculated. It is to be noted that conditions for the HPLC analysis were as follows:

Column: TSKgel G4000PWXL (product of Tosoh Corporation, 7.8 mm $\times$ 30.0 mm) $\times$ 2 columns
Flow rate: 1.0 mL/min
Detection: RI
Sample volume: 10 $\mu$L
Eluent: 0.2Npotassiumphosphatebuffer (pH: 6.88)
Pressure: 20 kg/cm$^2$
Temperature: 50°C

Example 1

Acid process gelatin (derived from pork skins, product of Sigma Chemicals) (10 g) was dissolved in water to give a total amount of 100 g, whereby a 10% aqueous solution of gelatin was prepared. In 15 cm $\times$ 15 cm trays coated at inner walls thereof with polytetrafluoroethylene, the thus-prepared aqueous solution of gelatin was poured in 30 g portions, respectively. The trays were placed in a drier (trade name: "Program Incubator IN800", manufactured by Yamato Scientific Co., Ltd.) set at 32°C, and the aqueous solution of gelatin poured in each tray was dried in air to obtain a gelatin film (3 g). After the gelatin film was placed in water chilled at 5°C and was hydrated there, $\gamma$ rays were irradiated to 20 kGy to subject the gelatin film to water insolubilization treatment. The swelling degree of the gelatin film subjected to the water insolubilization treatment was 600%, and its water elution rate was 3.5%. The gelatin film subjected to the water insolubilization treatment was immersed three times, each for 2 hours, in an aqueous solution of glycerol (concentration: 5%, 50 mL) so that the 5% aqueous solution of glycerol was held inside the gelatin film to obtain an adhesion preventing medical material. The content ratio of glycerol in the resultant adhesion preventing medical material was 25%, and its glycerol retention rate was 0%.

Example 2

Gelatin films (3 g each) were obtained as in Example 1 described above except that the temperature of the drier was set at 50°C. Each gelatin film so obtained was immersed in a 5 vol.% aqueous solution of glutaraldehyde (100 mL), and was reacted at room temperature for 6 hours to subject it to water insolubilization treatment. The swelling degree of the gelatin film subjected to the water insolubilization treatment was 550%, and its water elution rate was 3.6%. After the gelatin film subjected to the water insolubilization treatment was thoroughly washed with water, it was immersed three times, each for 2 hours, in an aqueous solution of glycerol (concentration: 5%, 50 mL) so that the 5% aqueous solution of glycerol was held inside the gelatin film to obtain an adhesion preventing medical material. The content ratio of glycerol in the resultant adhesion preventing medical material was 22.5%, and its glycerol retention rate was 0%.

Example 3

Gelatin films (3 g each) were obtained as in Example 1 described above. Each gelatin film so obtained was immersed in an ethanol solution (100 mL) with hexamethylene diisocyanate contained at 5 vol.%, and was reacted at room temperature for 18 hours to subject it to water insolubilization treatment. The swelling degree of the gelatin film subjected to the water insolubilization treatment was 450%, and its water elution rate was 4.0%. After the gelatin film subjected to the water insolubilization treatment was thoroughly washed with ethanol, it was immersed three times, each for 2 hours, in an aqueous solution of glycerol (concentration: 5%, 50mL) so that the 5% aqueous solution of glycerol was held inside the gelatin film to obtain an adhesion preventing medical material. The content ratio of glycerol in the resultant adhesion preventing medical material was 17.5%, and its glycerol retention rate was 0%.

Example 4

Sodium hyaluronate (molecular weight: 800,000, product of Shiseido Co., Ltd.) (1 g), glycerol ("Glycerin (Japanese Pharmacopoeia grade)", product of Sakamoto Yakuhin Kogyo Co., Ltd.)(0.5 g), and water were mixed to prepare a solution (100 mL in total) . In trays made of polytetrafluoroethylene (diameter: 13 cm), the thus-prepared solution was poured in 50 g portions, respectively. The solution poured in each tray was dried at 30°C in air to obtain a glycerol-containing film (1.3 g in total) . With the thus-obtained film being left in the tray, a 1% aqueous solution of $CaCl_2 \cdot 2H_2O$ (5 mL) was added, and the film was dried at 30°C in air, whereby the film was subjected to water insolubilization treatment to obtain an adhesion preventing medical material. The swelling degree of the film subjected to the water insolubilization treatment was 2,000%, and its water elution rate was 9%. The content ratio of glycerol in the resultant adhesion preventing medical material was 50%, and its glycerol retention rate was 0%.

Example 5

As in Example 4 described above, an aqueous sodium hyaluronate solution with glycerol contained therein was dried at 30°C in air to obtain a film (1.3 g in total) . Then, with the thus-obtained film being left in the tray, an aqueous titanium chelate agent (product name: "TC-315", product of Matsumoto Trading Co., Ltd.) was diluted tenfold in water, and subsequently, a portion (5 mL) of the thus-diluted solution was added to subject the filmtowater insolubilization. The swelling degree of the film subjected to the water insolubilization treatment was 1,800%, and its water elution rate was 2%. After the film subjected to the water insolubilization treatment was thoroughly washed with water, it was immersed three times, each for 2 hours, in an aqueous solution of glycerol (concentration: 5%, 50 mL) so that the 5% aqueous solution of glycerol was held inside the film to obtain an adhesion preventing medical material. The content ratio of glycerol in the resultant adhesion preventing medical material was 50%, and its glycerol retention rate was 0%.

Example 6

Carboxymethylcellulose (CMC) (product of Dai-ichi Kogyo Seiyaku Co., Ltd., "Cellogen PR-S Nikkyoku")(3 g) was added to water to prepare a CMC solution (100 g in total). The thus-prepared CMC solution was poured and dried in air as in Example 1 described above, so that CMC films were obtained. The resultant CMC films were placed in the incubator set at 120°C, whereby the CMC films were subjected to water insolubilization treatment by dry-heat crosslinking. The swelling degree of each water-insolubilized CMC film was 250%, and its water elution rate was 2%. The CMC film subjected to the water insolubilization treatment was immersed three times, each for 2 hours, in an aqueous solution of glycerol (concentration: 5%, 50 mL) so that the 5% aqueous solution of glycerol was held inside the CMC film to obtain an adhesion preventing medical material. The content ratio of glycerol in the resultant adhesion preventing medical material was 7.5%, and its glycerol retention rate was 0%.

Example 7

Sodium hyaluronate (molecular weight: 800,000, product of Shiseido Co., Ltd.) (1 g), glycerol ("Glycerin (Japanese Pharmacopoeia grade)", product of Sakamoto Yakuhin Kogyo Co., Ltd.)(0.5 g), and water were mixed to prepare a solution (100 mL in total) . In trays made of polytetrafluoroethylene (diameter: 13 cm), the thus-prepared solution was poured in 50 g portions, respectively. The solution poured in each tray was dried at 30°C in air to obtain a film with the aqueous glycerol solution contained therein (1.5 g in total). With the thus-obtained film being left in the tray, a 0.2 mol/L aqueous solution of hydrochloric acid (5 mL) was then added, and the film was dried at 30°C in air, whereby the film was subjected to water insolubilization treatment to obtain an adhesion preventing medical material. The swelling degree of the film subjected to the water insolubilization treatment was 1,000%, and its water elution rate was 10%. The content ratio of glycerol in the resultant adhesion preventing medical material was 10%, and its glycerol retention rate was 0%.

Example 8

Sodium hyaluronate (molecular weight: 800,000, product of Shiseido Co., Ltd.) (1 g), glycerol ("Glycerin (Japanese Pharmacopoeia grade)", product of Sakamoto Yakuhin Kogyo Co., Ltd.)(0.5 g), and water were mixed to prepare a solution (100 mL in total) . In trays made of polytetrafluoroethylene (diameter: 13 cm), the thus-prepared solution was poured in 50 g portions, respectively. The solution poured in each tray was dried at 30°C in air to obtain a film with the aqueous glycerol solution contained therein (1.5 g in total). With the thus-obtained film being left in the tray, a 0.2 mol/L aqueous solution of hydrochloric acid (5 mL) was then added, and the film was dried at 30°C in air, whereby the film was subjected to water insolubilization treatment. The film was then heated under reduced pressure at 120°C for 14 hours to obtain an adhesion preventing medical material. The swelling degree of the film subjected to the water insolubilization treatment was 900%, and its water elution rate was 6%. The content ratio of glycerol in the resultant adhesion preventing medical material was 10%, and its glycerol retention rate was 0%.

Comparative Example 1

Gelatin films subjected to water insolubilization treatment were obtained as in Example 1 described above except that the water insolubilization treatment was conducted by irradiating $\gamma$ rays to 5 kGy. The swelling degree of each gelatin film subjected to water insolubilization treatment was 3,500%, and its water elution rate was 60%. The gelatin film subjected to the water insolubilization treatment was then immersed three times, each for 2 hours, in an aqueous solution of glycerol (concentration: 5%, 50 mL) so that the 5% aqueous solution of glycerol was held inside the gelatin film. Subsequently, the film was dried in air to obtain a dry adhesion preventing medical material. The content ratio of glycerol in the resultant adhesion preventing medical material was 25%, and its glycerol retention rate was 0%.

Comparative Example 2

An adhesion preventing medical material, which used sodium hyaluronate as a bioresorbable material, was obtained as in Example 4 described above except that water insolubilization treatment was conducted using a 0.5% aqueous solution of $CaCl_2 \cdot 2H_2O$ (2 mL). The swelling degree of the film subjected to the water insolubilization treatment was 5,000%, and its water elution rate was 80%. The content ratio of glycerol in the resultant adhesion preventing medical material was 25%, and its glycerol retention rate was 0%.

Evaluation

Adult dogs (beagle, female, 1.5 years old) were subjected to thoracotomy after general anesthetic treatment, and their left lungs were exposed. Subsequent to $\gamma$ ray sterilization, the adhesion preventing medical materials (films) obtained in Examples 1 to 8 were arranged on surfaces of the lungs, respectively, followed by chest closure. Two weeks later, those dogs were subjected to general anesthetic treatment and then to thoracotomy. No adhesions were observed. Further, the films had been degraded. With respect to dogs subjected to similar treatment without the arrangement of such a film, on the other hand, significant adhesions were observed at their lungs and pleurae.
When similar treatment was performed using the adhesion preventing medical materials (films) obtained in Comparative Examples 1 and 2, adhesions were observed. A summary of the examples and comparative examples is shown in Table 1.

Table 1

| | Bioresorbable material | Polyhydric alcohol | Swelling degree (%) | Water elution rate (%) | Glycerol content ratio (%) | Glycerol retention rate (%) | Evaluation |
|---|---|---|---|---|---|---|---|
| Ex. 1 | Gelatin | Glycerol | 600 | 3.5 | 25 | 0 | No adhesions |
| Ex. 2 | Gelatin | Glycerol | 550 | 3.6 | 22.5 | 0 | |
| Ex. 3 | Gelatin | Glycerol | 450 | 4 | 17.5 | 0 | |
| Ex. 4 | Na hyaluronate | Glycerol | 2,000 | 9 | 50 | 0 | |
| Ex. 5 | Na hyaluronate | Glycerol | 1,800 | 2 | 50 | 0 | |
| Ex. 6 | CMC | Glycerol | 250 | 2 | 7.5 | 0 | |
| Ex. 7 | Na hyaluronate | Glycerol | 1,000 | 10 | 10 | 0 | |
| Ex. 8 | Na hyaluronate | Glycerol | 900 | 6 | 10 | 0 | |
| Comp. Ex. 1 | Gelatin | Glycerol | 3,500 | 60 | 25 | 0 | Adhered |
| Comp. Ex. 2 | Na hyaluronate | Glycerol | 5,000 | 80 | 25 | 0 | Adhered |

**Industrial Applicability**

The use of the adhesion preventing medical material according to the present invention can safely and surely prevent post-operative adhesions at various tissues and sites.

**Claims**

1. An adhesion preventing medical material comprising a bioresorbable base material and an aqueous polyhydric alcohol solution, which contains the polyhydric alcohol, held in said bioresorbable base material, said bioresorbable base material comprising a bioresorbable material and having a swelling degree of 200 to 3,000 mass% and a water elution rate of not higher than 10 mass%,

   wherein, after immersed for 3 hours in water of 25°C in an amount at least 50 times a total mass of the aqueous polyhydric alcohol solution and the bioresorbable base material, the polyhydric alcohol remains in an amount of not greater than 30 mass% of that of the polyhydric alcohol before the immersion,
   wherein the bioresorbable material is at least one bioresorbable material selected from the group consisting of gelatin, collagen, chitin, partially-deacetylated chitin, chitosan, hyaluronic acid, carboxymethylcellulose, derivatives thereof, and salts thereof,
   wherein the proportion of the polyhydric alcohol (the content ratio of the polyhydric alcohol) to the mass of the bioresorbable base material is 10 mass% or greater, and
   wherein the initial concentration of a polyhydric alcohol, namely the concentration of an aqueous solution of the polyhydric alcohol contained in the adhesion preventing medical material is in a range of 1 to 20 wt%.

2. The adhesion preventing medical material according to claim 1, wherein the polyhydric alcohol is glycerol, and the glycerol amounts to a proportion of not greater than 70 mass% of a mass of the bioresorbable base material.

3. The adhesion preventing medical material according to any one of claims 1 or 2, wherein the bioresorbable base material is in a form of a film, spheres, a string, a rod, a plate, a tube, rectangles, a powder, a colloid, a liquid or a sponge.

4. The adhesion preventing medical material according to any one of claims 1-3, which has an in vivo shape retention time (in days) (T) of 1<T<21.

5. A production process of the adhesion preventing medical material according to any one of claims 1-4, comprising the following step:
holding, in the bioresorbable base material, the aqueous polyhydric alcohol solution with the polyhydric alcohol contained therein.

6. The production process according to claim 5, wherein the aqueous polyhydric alcohol solution is held after subjecting the bioresorbable base material to water insolubilization treatment.

7. The production process according to claim 5, wherein the bioresorbable base material with the polyhydric alcohol contained therein is subjected to water insolubilization treatment.

8. The production process according to claim 5, wherein, when the bioresorbable material is a salt that dissolves in a neutral range, the bioresorbable base material is subjected to water insolubilization treatment by adjusting its pH before or after the adhesion preventing medical material is processed into a predetermined form.

9. The production process according to claim 6, wherein the bioresorbable base material is subjected to water insolubilization treatment by irradiation of radiation.

10. The production process according to claim 9, wherein the radiation is irradiated to 10 to 60 kGy.

11. The production process according to claim 6 or 8, wherein the bioresorbable base material is subjected to water insolubilization treatment with polyvalent cations.

12. The production process according to claim 11, wherein the polyvalent cations are at least one type of polyvalent cations selected from a group consisting of calcium ions, titanium ions, magnesium ions, iron ions, and zirconium ions.

13. The production process according to claim 6 or 7, wherein the bioresorbable base material is subjected to water insolubilization treatment with a compound that has at least two functional groups in a molecule thereof.

14. The production process according to any one of claims 5-13, further comprising the following step:
lyophilizing an aqueous solution of the bioresorbable material to obtain the bioresorbable base material in a sponge form.

**Patentansprüche**

1. Adhäsion-verhinderndes medizinisches Material, umfassend ein bioresobierbares Ausgangsmaterial und eine wässrige, mehrwertige Alkohollösung, die einen mehrwertigen Alkohol enthält, die in dem Ausgangsmaterial gehalten wird, wobei das bioresobierbare Ausgangsmaterial ein bioresobierbares Material umfasst und einen Quellungsgrad von 200 bis 3000 Massenprozent und eine Elutionsrate von Wasser von nicht höher als 10 Massenprozent aufweist; wobei, der mehrwertige Alkohol, nachdem er für 3 Stunden in Wasser von 25°C in einer Menge von wenigstens 50-mal der Gesamtmasse der wässrigen mehrwertigen Alkohollösung und des bioresobierbaren Ausgangsmaterials eingetaucht wurde, in einer Menge von nicht mehr als 30 Massenprozent von der des mehrwertigen Alkohols vor dem Eintauchen verbleibt, wobei das bioresobierbare Material wenigstens ein bioresobierbares Material ist, ausgewählt aus der Gruppe, bestehend aus Gelatine, Kollagen, Chitin, teilweise deacetyliertem Chitin, Chitosan, Hyaluronsäure, Carboxymethylcellulose, Derivaten derselben und Salzen derselben, wobei der Anteil des mehrwertigen Alkohols (Gehaltsanteil des mehrwertigen Alkohols) zu der Masse des bioresobierbaren Ausgangsmaterials 10 Massenprozent oder mehr beträgt, und wobei die Anfangskonzentration des mehrwertigen Alkohols, und zwar die Konzentration einer wässrigen Lösung des mehrwertigen Alkohols, der in dem Adhäsion-verhindernden medizinischen Material enthalten ist, in einem Bereich von 1 bis 20 Gewichtsprozent liegt.

2. Adhäsion-verhinderndes medizinisches Material nach Anspruch 1, wobei der mehrwertige Alkohol Glycerol ist und sich das Glycerol auf einen Anteil von nicht mehr als 70 Massenprozent der Masse des bioresobierbaren Ausgangsmaterials beläuft.

**3.** Adhäsion-verhinderndes medizinisches Material nach einem der Ansprüche 1 oder 2, wobei das bioresobierbare Ausgangsmaterial in Form eines Films, von Kugeln, eines Strangs, eines Stabs, einer Platte, eines Röhrchens, von Rechtecken, eines Pulvers, eines Kolloids, einer Flüssigkeit oder eines Schwamms vorliegt.

**4.** Adhäsion-verhinderndes medizinisches Material nach einem der Ansprüche 1 bis 3, welches *in vivo* eine Dauer der Formbeständigkeit (in Tagen) (T) von 1 < T < 21 aufweist.

**5.** Herstellungsverfahren für das Adhäsion-verhindernde medizinische Material nach einem der Ansprüche 1 bis 4, umfassend den folgenden Schritt:
Halten, in dem bioresobierbaren Ausgangsmaterial, der wässrigen mehrwertigen Alkohollösung mit dem darin enthaltenen mehrwertigen Alkohol.

**6.** Herstellungsverfahren nach Anspruch 5, wobei die wässrige mehrwertige Alkohollösung gehalten wird, nachdem das bioresobierbare Ausgangsmaterial einer Behandlung zur Insolubilisierung in Wasser unterzogen wurde.

**7.** Herstellungsverfahren nach Anspruch 5, wobei das bioresobierbare Ausgangsmaterial mit dem darin enthaltenen mehrwertigen Alkohol einer Behandlung zur Insolubilisierung in Wasser unterzogen wird.

**8.** Herstellungsverfahren nach Anspruch 5, wobei, wenn das bioresobierbare Material ein Salz ist, das sich in einem neutralen Bereich löst, das bioresobierbare Ausgangsmaterial einer Behandlung zur Isolubilisierung in Wasser unterzogen wird, indem dessen pH eingestellt wird, bevor oder nachdem das Adhäsion-verhindernde medizinische Material zu einer vorbestimmten Form verarbeitet wird.

**9.** Herstellungsverfahren nach Anspruch 6, wobei das bioresobierbare Ausgangsmaterial der Behandlung zur Insolubilisierung in Wasser durch Bestrahlung mit einer Strahlung unterzogen wird.

**10.** Herstellungsverfahren nach Anspruch 9, wobei mit einer Strahlung von 10 bis 60 kGy bestrahlt wird.

**11.** Herstellungsverfahren nach Anspruch 6 oder 8, wobei das bioresobierbare Ausgangsmaterial der Behandlung zur Insolubilisierung in Wasser mit mehrwertigen Kationen unterzogen wird.

**12.** Herstellungsverfahren nach Anspruch 11, wobei die mehrwertigen Kationen wenigstens eine Art von mehrwertigen Kationen sind, ausgewählt aus der Gruppe, bestehend aus Calcium-Ionen, Titan-Ionen, Magnesium-Ionen, Eisen-Ionen und Zirkonium-Ionen.

**13.** Herstellungsverfahren nach Anspruch 6 oder 7, wobei das bioresobierbare Ausgangsmaterial der Behandlung zur Insolubilisierung in Wasser mit einer Verbindung unterzogen wird, die wenigstens zwei funktionelle Gruppen in ihrem Molekül aufweist.

**14.** Herstellungsverfahren nach einem der Ansprüche 5 bis 13, ferner den folgenden Schritt umfassend:
Gefriertrocknen einer wässrigen Lösung des bioresobierbaren Materials, um das bioresobierbare Ausgangsmaterial in Form eines Schwamms zu erhalten.

## Revendications

**1.** Matériau médical empêchant une adhérence, comprenant un matériau de base biorésorbable et une solution aqueuse d'alcool polyvalent, qui contient l'alcool polyvalent, retenu dans ledit matériau de base biorésorbable, ledit matériau de base biorésorbable comprenant un matériau biorésorbable et présentant un degré de gonflement de 200 à 3000 % en masse et un taux d'élution de l'eau non supérieur à 10 % en masse,
dans lequel, après 3 heures d'immersion dans de l'eau à 25°C en une quantité d'au moins 50 fois la masse totale de la solution aqueuse d'alcool polyvalent et du matériau de base biorésorbable, la quantité résiduelle d'alcool polyvalent n'est pas supérieure à 30 % en masse de celle de l'alcool polyvalent avant l'immersion,
dans lequel le matériau biorésorbable est au moins un matériau biorésorbable choisi dans l'ensemble constitué par la gélatine, le collagène, la chitine, la chitine partiellement désacétylée, le chitosane, l'acide hyaluronique, la carboxyméthylcellulose, leurs dérivés, et leurs sels,

dans lequel la proportion de l'alcool polyvalent (le rapport en contenu de l'alcool polyvalent) par rapport à la masse du matériau de base biorésorbable est de 10 % en masse ou plus, et

dans lequel la concentration initiale de l'alcool polyvalent, à savoir la concentration de la solution aqueuse de l'alcool polyvalent se trouvant dans le matériau médical empêchant une adhérence, est située dans la plage allant de 1 à 20 % en poids.

2. Matériau médical empêchant une adhérence selon la revendication 1, dans lequel l'alcool polyvalent est le glycérol, et la quantité de glycérol correspond à une proportion non supérieure à 70 % en masse de la masse du matériau de base biorésorbable.

3. Matériau médical empêchant une adhérence selon l'une quelconque des revendications 1 et 2, dans lequel le matériau de base biorésorbable est sous la forme d'un film, de sphères, d'un fil, d'une tige, d'une plaque, d'un tube, d'un rectangle, d'une poudre, d'un colloïde, d'un liquide ou d'une éponge.

4. Matériau médical empêchant une adhérence selon l'une quelconque des revendications 1 à 3, qui a un temps de maintien de forme in vivo (en jours) (T) satisfaisant à 1 < T < 21.

5. Procédé de production du matériau médical empêchant une adhérence selon l'une quelconque des revendications 1 à 4, comprenant l'étape suivante :

maintien, dans le matériau de base biorésorbable, de la solution aqueuse d'alcool polyvalent, avec l'alcool polyvalent contenu dans celui-ci.

6. Procédé de production selon la revendication 5, dans lequel la solution aqueuse d'alcool polyvalent est maintenue après que le matériau de base biorésorbable a été soumis à un traitement d'insolubilisation dans l'eau.

7. Procédé de production selon la revendication 5, dans lequel le matériau de base biorésorbable avec l'alcool polyvalent contenu dans celui-ci est soumis à un traitement d'insolubilisation dans l'eau.

8. Procédé de production selon la revendication 5, dans lequel, quand le matériau biorésorbable est un sel qui se dissout dans une plage neutre, le matériau de base biorésorbable est soumis à un traitement d'insolubilisation dans l'eau par ajustement de son pH avant ou après mise du matériau médical empêchant une adhérence sous une forme prédéterminée.

9. Procédé de production selon la revendication 6, dans lequel le matériau de base biorésorbable est soumis à un traitement d'insolubilisation dans l'eau par irradiation d'un rayonnement.

10. Procédé de production selon la revendication 9, dans lequel le rayonnement est irradié à 10 à 60 kGy.

11. Procédé de production selon la revendication 6 ou 8, dans lequel le matériau de base biorésorbable est soumis à un traitement d'insolubilisation dans l'eau avec des cations polyvalents.

12. Procédé de production selon la revendication 11, dans lequel les cations polyvalents sont au moins un type de cations polyvalents choisis dans l'ensemble constitué par les ions calcium, les ions titane, les ions magnésium, les ions fer, et les ions zirconium.

13. Procédé de production selon la revendication 6 ou 7, dans lequel le matériau de base biorésorbable est soumis à un traitement d'insolubilisation dans l'eau avec un composé qui possède au moins deux groupes fonctionnels par molécule.

14. Procédé de production selon l'une quelconque des revendications 5 à 13, comprenant en outre l'étape suivante :

lyophilisation d'une solution aqueuse du matériau biorésorbable pour que soit obtenu le matériau de base biorésorbable sous la forme d'une éponge.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2008155014 A
- JP 2006231090 A
- JP 2000037450 A
- JP 2010213984 A

### Non-patent literature cited in the description

- **FUJISHITA, AKIRA ; YOSHIDA, SHIKO ; SIMOMURA, TOMOKO ; MATSUMOTO, AYUMI.** An Overview of Adhesion Preventing Methods and Adhesion Preventing Measures - Centering around Gynecology-related Literature. *OBSTERICAL AND GYNECOLOGICAL PRACTICE,* 2010, vol. 59 (8), 1159-1167
- **SUGIHARA, HISASHI.** Erythrocyte Hemolysis by Glycerol. *THE JAPANESE JOURNAL of CLINICAL HEMATOLOGY,* 1983, vol. 24 (8), 1012-1019
- **TAKEDA, TOSHIAKI ; ISHIDA, YOKO ; KAWASHIMA, MIDORI.** Experimental Study on Relationship between Glycerol Enema Solution and Hemolysis in Rats. *JOURNAL OF JAPANESE SOCIETY OF NURSING RESEARCH,* 2003, vol. 26 (4), 81-88
- **TAKEDA, TOSHIAKI.** Verification Study on Induction of Hemolysis by Glycerol Enema in Experimental Animals. *JAPANESE JOURNAL OF NURSING ART AND SCIENCE,* 2006, vol. 5 (1), 45-50
- **YASUHARU NOISHIKI et al.** Anti-adhesive Membrane for Pleural Cavity. *Artificial Organs,* 2010, vol. 32 (3), 224-229
- Program Incubator IN800. Yamato Scientific Co., Ltd,